# EUROPEAN PATENT APPLICATION

(11) **EP 4 296 366 A1**
(43) Date of publication of application: **27.12.2023**
(21) Application number: 21952650.6
(22) Date of filing: 29.12.2021
(51) Int. Cl.: C12P 7/62, C12P 39/00, C12R 1/46, C12R 1/25, C12R 1/23, C12R 1/225, C12R 1/01

(54) **METHOD FOR PRODUCING POLYHYDROXYALKANOATE BY FERMENTATION OF AGRICULTURAL WASTES**

(30) Priority: 02.08.2021 CN 202110878525
(71) Applicant: Institute of Environment and Sustainable Development in Agriculture Chinese Academy of Agricultural Sciences, Beijing 100081 (CN)
(72) Inventor: DONG, Hongmin, Beijing 100081 (CN); WANG, Shunli, Beijing 100081 (CN); ZHANG, Wanqin, Beijing 100081 (CN); YIN, Fubin, Beijing 100081 (CN); CAO, Qitao, Beijing 100081 (CN); LIAN, Tianjing, Beijing 100081 (CN); ZHOU, Tanlong, Beijing 100081 (CN); ZHU, Zhiping, Beijing 100081 (CN)
(74) Representative: Slingsby Partners LLP
(86) International application number: PCT/CN2021/142357
(87) International publication number: WO 2023/010787

(57) **Abstract**

The present invention provides a method for producing a polyhydroxyalkanoate (PHA) by fermentation of agricultural wastes. In the present invention, a fermentation substrate is agricultural wastes, comprising a mixture of livestock and poultry manure and a planting industry waste. The method comprises: at a primary fermentation stage, a zymophyte is a lactic acid bacterium, a fermentation temperature is 35-55°C, a pH value is 3.0 to 6.0, and oxidation-reduction potential in a system is maintained at -250 to -450 mV by means of intermittent aeration; and at a secondary fermentation stage: aerobic fermentation is performed by using acclimated sludge, a fermentation temperature is 20-40°C, and a pH value is 7.0-9.0. According to the present invention, under the condition that an exogenous carbon source and an exogenous nitrogen source are not additionally added, an organic carbon source in the livestock and poultry manure and the planting industry waste is efficiently converted into the PHA, and thus, the production source of the PHA can be broadened, the production cost of the PHA can be reduced, and the potential pollution of the livestock and poultry manure and the planting industry waste is reduced.

## Description

### Cross-reference to related application

The present application claims priority to Chinese Patent Application No. 202110878525.5, entitled "Method for producing polyhydroxyalkanoate by fermentation of agricultural wastes", and filed on August 2, 2021, the entire disclosure of which is incorporated herein by reference in its entirety.

### Technical Field

The present application belongs to the field of agricultural waste utilization, and specifically relates to a method for producing polyhydroxyalkanoate (PHA) by fermentation of agricultural wastes.

### Background Art

Polyhydroxyalkanoate (PHA) is a new type of biodegradable plastic material that exhibits similar material properties to traditional plastic products. Different from traditional plastics, PHA can be completely degraded into CO₂/CH₄ and water under natural environments (such as soil and seawater) or biological treatment (such as anaerobic digestion and aerobic composting), which is an environmentally friendly biodegradable material. The promotion and application of PHA is an effective way to solve white pollution of plastic and improve the ecological environment, and the efficient production and application of PHA have also received widespread attention.

The current PHA fermentation process mainly uses glucose, vegetable oil and the like as carbon sources for synthesis under imbalance conditions of high carbon and low nitrogen. The high cost of raw materials has limitted large-scale production and application of PHA. In order to reduce production costs, scholars have studied a series of methods for producing PHA from waste biomass. For example, Chinese patent No. CN111394398A discloses a method for fermenting high salt molasses as raw materials to manufacture PHA, which reduces the cost of carbon source by replacing glucose with molasses wastewater; CN104357496B discloses a method for synthesizing PHA using corn slurry hydrolysate, which uses hydrolyzed corn slurry to provide a nitrogen source and reduces cost of adding nitrogen sources in PHA synthesis; and CN111892699A discloses equipment and method for synthesizing PHA from kitchen waste, which firstly acidifies kitchen waste and then synthesizes PHA. Compared to kitchen waste, livestock and poultry manure is of large quantity with stable characteristics, but its N or P content is higher, which can easily lead to nutrient balance in the PHA cultivation process, resulting in conversion and utilization of PHA by microorganisms, which is not conducive to accumulation of PHA. This makes it more difficult to produce PHA using livestock and poultry manure compared to other materials, and there is currently no ideal method.

In view of this, it is necessary to explore a new and more scalable method for synthesizing PHA from waste containing complex components such as high nitrogen, phosphorus, organic matter, and minerals, as a raw material.

### Summary of the Invention

The present application provides a two-stage fermentation method for producing PHA using agricultural wastes such as livestock and poultry manure to solve high cost of the raw material in PHA production process.

Specifically, technical solutions of the present application are as follows:
A method for producing polyhydroxyalkanoate (PHA) by fermentation of agricultural waste, wherein the fermentation substrate is agricultural waste, the agricultural waste comprises a mixture of livestock and poultry manure and a planting industry waste, and the planting industry waste is crop straw and/or fruit and vegetable waste; the method comprises:
A. a primary fermentation stage, which is performed with zymophyte of lactic acid bacteria, at a fermentation temperature of 35 to 55 °C, a pH of 3.0 to 6.0, and an oxidation-reduction potential (ORP) in the fermentation system maintained at -250 mV to -450 mV by means of intermittent air aeration; and
B. a secondary fermentation stage, wherein aerobic fermentation is performed by using acclimated sludge at a fermentation temperature of 20 to 40 °C and a pH of 7.0 to 9.0.

On the basis of existing PHA synthesis technology research, the present application proposes a solution for producing PHA using livestock and poultry manure and other agricultural wastes from planting industry sources as raw materials through a two-stage fermentation process. The main components of PHA are PHB and PHV

Specifically, in the present application, different functional bacterial strains are selected for each stage, in combination with control of specific conditions in each fermentation stage, so as to enable the targeted generation of lactic acid and various volatile fatty acids with specific proportions at a primary fermentation stage, thereby achieving efficient production of PHA at a subsequent secondary fermentation stage.

Preferably, at a primary fermentation stage, the fermentation temperature is 35 to 45 °C and the pH is 4.5 to 5.5. This condition can enhance the substrate competitiveness of dominant lactic acid-producing bacteria, while the lactic acid produced by fermentation will lower pH of the system, and further inhibit the metabolic activity of other microorganisms, and then promote the enrichment of dominant lactic acid-producing bacteria, which is conducive to achieving targeted conversion of organic carbon sources in agricultural wastes such as manure to specific proportions of lactic acid and various volatile fatty acids.

The volatile fatty acid in the present application is acetic acid, propionic acid, butyric acid and the like (there may also be a small amount of isobutyric acid, valeric acid, isovaleric acid, caproic acid and the like).

Preferably, the secondary fermentation stage is performed at a fermentation temperature of 22 to 28 °C and a pH of 7.5 to 8.5 to improve the utilization efficiency of organic carbon sources such as lactic acid by PHA synthesis bacteria.

In the present application, the fermentation substrate is a mixture of livestock and poultry manure and fruit and vegetable waste, with a carbon to nitrogen ratio of the mixture of (30 to 50): 1, preferably (35 to 40): 1;

Alternatively, the fermentation substrate is a mixture of livestock and poultry manure and crop straw, with a carbon to nitrogen ratio of the mixture of (30 to 40): 1.

In the present application, it is found that when the proportion of livestock and poultry manure and a planting industry waste in the fermentation substrate is further controlled to make it at a carbon to nitrogen ratio defined by the present application, it can be conducive to the targeted conversion of organic carbon source in the waste to a specific proportion of lactic acid and a variety of volatile fatty acids, and by combining with the content of N, P and other trace elements in the fermentation broth, the production of PHA at the secondary fermentation stage is thus ensured.

The fruit and vegetable wastes of the present application are rotten fruits, seedlings, vines, leaves produced in the process of planting fruits or vegetables, and tailings in the processing. Crop straw is one or more of corn straw, wheat straw, and rice straw. In the fruit and vegetable waste, total solids (TS) are 2% to 20%, volatile solids (VS) are 90% to 98% TS, total sugar content is 10 to 20% (based on fresh weight), and carbon to nitrogen ratio is (40 to 100): 1. In the crop straw, TS are 30% to 70%, VS are 85% to 95% TS, crude fiber content is 35% to 55%, and carbon to nitrogen ratio is (45 to 60): 1.

In the present application, as a specific embodiment, the air aeration rate at the primary fermentation stage is 0 to 2000 mL/L/d.

Preferably, the specific air aeration intensity at a primary fermentation stage is 0 to 400 mL/L/min, and the aeration time is 0 to 10 min/d.

In the present application, the aerobic fermentation with continuous air aeration is performed at a secondary fermentation stage with an aeration intensity of 0.8 to 1.2 L/L/min.

In the present application, the lactic acid bacterium comprises one or more of *Bifidobacterium, Streptococcus thermophilus, Lactobacillus plantarum, Lactobacillus acidophilus, Lactobacillus rhamnosus,* and *Lactobacillus bulgaricus.*

In the present application, preparation method of the acclimated sludge is as follows:
the bottom sludge of aerobic aeration tank in the sewage treatment plant is acclimated in a fermentation system with a continuous air aeration, and a pH of 6.5 to 9.5 at room temperature (20 to 30 °C), by using acetic acid as a carbon source and ammonium chloride as a nitrogen source;
and/or, the intensity of continuous air aeration is 0.8 to 1.2 L/L/min.

The main microbial species in the acclimated sludge are bacteria that can synthesize PHA. The general acclimation time is about 4 months, based on achieving goal of enriching the flora that can produce PHA efficiently.

Preferably, after acclimation, the main flora in the acclimated sludge is *Paracoccus* and *Gemmobacter,* and the content of PHA accounts for 27% to 42% dry weight of bacterial cells.

In the present application, the fermentation concentration at the primary fermentation stage is 2% to 15% VS; the inoculation amount of the bacterial broth is 2% to 10% (V/V), and the number of live bacteria in the bacterial broth is 10⁶ to 10⁹ CFU/mL;
and/or, the fermentation concentration at the secondary fermentation stage is 60 to 120 mmol C/L, preferably 100 to 120 mmol C/L; the inoculation amount of acclimated sludge is 40% to 80% (V/V), and a hydraulic retention time (HRT) is 5 to 36 hours. The above conditions complement each other to ensure the final fermentation effect.

The beneficial effects of the present application are as follows.

The present application provides a method for producing PHA through two-stage fermentation by using livestock and poultry manure and other agricultural wastes. The yield of PHA is high, and the fermentation substrate is livestock and poultry manure, crop straw, fruit and vegetable waste and the like, without adding additional exogenous carbon source and nitrogen sources. The raw material source is widely available, expanding the substrate source of PHA and significantly reducing the raw material cost of PHA production process. At the same time, the present application has also achieved harmless treatment and high-value utilization of agricultural organic wastes such as livestock and poultry manure, with good application prospects.

### Specific Modes for Carrying Out the Embodiments

The follows will provide a detailed explanation of preferred embodiments of the present application in conjunction with examples. It should be understood that the following examples are provided for illustrative purposes only and are not intended to limit the scope of the present application. A person skilled in the art may make various modifications and replacements to the present application without departing from its purpose and spirit.

In the specific implementation of the present application, the contents of lactic acid and volatile fatty acids are measured using a liquid chromatograph and a gas chromatograph, respectively. The specific instrument configuration and measurement method are shown in: Lian T, Zhang W, Cao Q, et al. Enhanced lactic acid production from the anaerobic co-digestion of swine manure with apple or potato waste via ratio adjustment [J]. Bioresource Technology, 2020, 318: 124237.

The PHA content is measured using a gas chromatograph, and the specific instrument configuration and measurement method are shown in: Pinto-Ibieta F, et al. Strategy for biological co-production of levulinic acid and polyhydroxyalkanoates by using mixed microbial cultures fed with synthetic hemicellulose hydrolysate [J]. Bioresource Technology, 2020, 309: 123323.

### Example 1: Production of PHA by mixed fermentation of pig manure and fruit and vegetable

The present Example comprises the following steps:
1. Lactic acid bacteria cultivation: a culture medium of lactic acid bacteria - MRS broth (Qingdao High tech Industrial Park Haibo Biotechnology Co., Ltd.) was prepared, Baishengyou lactic acid bacteria powder (4 g/L, purchased from Thankcome Biotechnology (Suzhou) Co., Ltd.) was inoculated, and cultivated for 36 hours under a controlled cultivation temperature of 35 to 45 °C and pH of 6.0 to 7.0, respectively.
2. The cultured lactic acid bacteria broth (with the number of live bacteria of approximately 10⁷ CFU/mL) was inoculated into an anaerobic fermentation reactor with pig manure and apple waste as substrates. The carbon to nitrogen ratio of the mixed substrate was (35 to 40): 1, and the inoculation amount was 10% (V/V). The fermentation concentration was 4% VS, the pH was controlled at 4.5 to 5.5, and the fermentation temperature was controlled at 35 to 40 °C. The oxidation-reduction potential (ORP) in the environment (fermentation system) was maintained at -250 mV to -450 mV by means of intermittent aeration with a specific intensity of air aeration of 200 mL/L/min and an air aeration time of 5 min/d. After 2 weeks of continuous anaerobic fermentation, the total carboxylic acid yield, lactic acid yield, and volatile fatty acid yield of the substrate were 932.6, 880.3, and 52.3 mg/g VS, respectively. Among the volatile fatty acids, the yields of acetic acid, propionic acid, and butyric acid were 16.6, 1.6, and 25.9 mg/g VS, respectively.
3. Sludge acclimation: the bottom sludge of aerobic aeration tank in the sewage treatment plant as the initial source of PHA bacteria was acclimated for 4 months with a continuous air aeration, and a pH of 6.8 to 9.3 at room temperature (20 to 30 °C), by using acetic acid as a carbon source and ammonium chloride as a nitrogen source. After acclimation in the present application, the dominant PHA synthesis bacteria in the sludge were *Paracoccus* (30%) and *Gemmobacter* (36%) with PHA content accounting for 27% to 42% of dry weight of bacterial cells.
4. The acidified broth obtained from the fermentation in step 2 was used as the raw material, and was inoculated with the acclimated sludge in step 3 at an initial inoculation amount of 70% (V/V), and no further supplementation will be required during subsequent continuous operation. The fermentation concentration was 108 mmol C/L, pH was controlled between 7.8 and 8.3, the fermentation temperature was controlled between 22 °C and 28 °C, the continuous air aeration was 0.8 L/L/min, and HRT was controlled at 24 hours. After 3 weeks of continuous fermentation, PHA concentration in the system reached 328 mg/L, and the yield was 348 mg PHA/g VS in which the specific components comprise PHB (97%) and PHV (3%).

### Example 2: Production of PHA by mixed fermentation of pig manure and straw

The production steps of the present Example were the same as Example 1, except that in Step 2, the raw materials for fermentation were pig manure and corn straw waste, and the carbon to nitrogen ratio of the mixed substrate was 35:1. After 2 weeks of continuous anaerobic acidification fermentation, the total carboxylic acid yield, lactic acid yield, and volatile fatty acid yield were 640.8, 609.4, and 31.4 mg/g VS, respectively. Among the volatile fatty acids, the yields of acetic acid and butyric acid were 14.4 and 17.0 mg/g VS, respectively.

The acidified broth was subjected to two-stage aerobic fermentation (process parameters are the same as steps 3 and 4 in Example 1), and after 3 weeks of continuous fermentation, PHA concentration in the system reached 314 mg/L, with a yield of 326 mg PHA/g VS, in which the specific components comprise PHB (96.9%) and PHV (3.1%).

### Example 3

The production steps of the present Example were the same as Example 1, except that in Step 2, the pH of fermentation was controlled at 3.5 and the fermentation temperature was controlled at 55 °C.

Primary fermentation results: after 2 weeks of continuous anaerobic fermentation, the total carboxylic acid yield, lactic acid yield, and volatile fatty acid yield of the material were 373.4, 342.5, and 30.9 mg/g VS, respectively. Among the volatile fatty acids, the yields of acetic acid, propionic acid, and butyric acid were 5.0, 2.7, and 23.2 mg/g VS, respectively.

Secondary fermentation results: after 3 weeks of continuous fermentation, PHA concentration in the system reached 277 mg/L, and the yield was 275 mg PHA/g VS, in which the specific components comprise PHB (96.5%) and PHV (3.5%).

### Example 4

The production steps of the present Example were the same as Example 1, except that in Step 4, the fermentation concentration was controlled at 60 mmol C/L. After 3 weeks of continuous fermentation, PHA concentration in the system reached 286 mg/L, and the yield was 217 mg PHA/g VS, in which the specific components comprise PHB (94%) and PHV (6%).

### Example 5

The production steps of the present Example were the same as Example 1, except that in Step 4, the fermentation temperature was controlled at 40 °C. After 3 weeks of continuous fermentation, PHA concentration in the system reached 252 mg/L, and the yield was 267 mg PHA/g VS, in which the specific components comprise PHB (95.5%) and PHV (4.5%).

### Comparative Example 1

Referring to the concentration of lactic acid in the acidified broth in Example 1, PHA was synthesized using an artificially prepared acidified broth containing only lactic acid as a carbon source. The conditions for synthesizing PHA were the same as those in Steps 3 and 4 of Example 1. After 3 weeks of continuous fermentation, PHA concentration in the system reached 157 mg/L, and the yield was 173 mg PHA/g VS, with the main component of PHB (99.4%). Compared to Example 1 and Example 2, the PHA yield decreased by 50.3% and 46.9%, respectively, indicating that the acidification broth of agricultural waste obtained by the present application has significant advantages in synthesizing PHA.

### Comparative Example 2

The production steps of the present Example were the same as Example 1, except that in Step 4, the HRT was controlled at 4 hours.

Secondary fermentation results: after 3 weeks of continuous fermentation, PHA concentration in the system reached 174 mg/L, and the yield was 106 mg PHA/g VS, in which the specific components comprise PHB (95.1%) and PHV (4.9%).

### Comparative Example 3

The production steps of the present Example were the same as Example 1, except that in Step 2, the pH of fermentation was controlled at 6.5 and the fermentation temperature was controlled at 60 °C.

Primary fermentation results: After 2 weeks of continuous anaerobic fermentation, the total carboxylic acid yield, lactic acid yield, and volatile fatty acid yield of the substrate were 192.7, 174.2, and 18.5 mg/g VS, respectively. Among the volatile fatty acid components, only butyric acid was detected, with a yield of 18.5 mg/g VS.

Secondary fermentation results: After 3 weeks of continuous fermentation, PHA concentration in the system reached 158 mg/L, and the yield was 106 mg PHA/g VS, in which the specific components comprise PHB (95.4%) and PHV (4.6%).

### Comparative Example 4

The production steps of the present Example were the same as Example 1, except that in Step 2, pig manure and apple waste were used as substrate, and the carbon to nitrogen ratio of the mixed substrate was 55:1.

Primary fermentation results: After 2 weeks of continuous anaerobic fermentation, the total carboxylic acid yield, lactic acid yield, and volatile fatty acid yield of the substrate were 127.4, 119.5, and 7.9 mg/g VS, respectively. Among the volatile fatty acid components, only butyric acid was detected, with a yield of 7.9 mg/g VS.

Secondary fermentation results: after 3 weeks of continuous fermentation, PHA concentration in the system reached 168 mg/L, and the yield was 120 mg PHA/g VS, in which the specific components comprise PHB (95.5%) and PHV (4.5%).

### Comparative Example 5

The production steps of the present Example were the same as Example 1, except that in Step 2, the ORP in the system was maintained at -220 mV by means of intermittent aeration, with a specific air aeration intensity of 500 mL/L/min and an aeration time of 10 minutes/d.

Primary fermentation results: after 2 weeks of continuous anaerobic fermentation, the total carboxylic acid yield, lactic acid yield, and volatile fatty acid yield of the substrate were 320.6, 150.8, and 169.8 mg/g VS, respectively. Among the volatile fatty acids, the yields of acetic acid, propionic acid, and butyric acid were 85.2, 33.6, and 51.0 mg/g VS, respectively.

Secondary fermentation results: after 3 weeks of continuous fermentation, PHA concentration in the system reached 162 mg/L, and the yield was 151 mg PHA/g VS, in which the specific components comprise PHB (94.7%) and PHV (5.3%).

Although the present application has been described in detail with general explanations and specific embodiments in the previous text, it is obvious to a person skilled in the art that some modifications or improvements can be made based on the present application. Therefore, these modifications or improvements made without deviating from the spirit of the present application belong to the protection scope claimed by the present application.

### Industrial applicability

The present application belongs to the field of agricultural waste utilization, and specifically relates to a method for producing a polyhydroxyalkanoate (PHA) by fermentation of agricultural wastes. In the present application, the fermentation substrate is agricultural waste, comprising the mixture of livestock and poultry manure and the planting industry waste. The method comprises the following steps: a primary fermentation stage, which is performed with zymophyte of lactic acid bacteria, at a fermentation temperature of 35 to 55 °C, a pH of 3.0 to 6.0, and an ORP in the system maintained at -250 mV to -450 mV by means of intermittent aeration; and a secondary fermentation stage, wherein aerobic fermentation is performed by using acclimated sludge at a fermentation temperature of 20 to 40 °C and a pH of 7.0 to 9.0. The present application efficiently converts organic carbon sources in livestock and poultry manure and a planting industry waste into PHA without adding additional exogenous carbon sources and nitrogen sources, which can not only broaden the production substrate source of PHA, reduce the production cost of PHA, but also reduce the potential pollution of livestock and poultry manure and planting industry waste, providing a new way for their high-value utilization.

## Claims

1. A method for producing a polyhydroxyalkanoate (PHA) by fermentation of agricultural wastes, wherein the polyhydroxyalkanoate comprises PHB and PHV; a fermentation substrate is agricultural wastes, the agricultural waste comprises a mixture of livestock and poultry manure and a planting industry waste, and the planting industry waste is crop straw and/or fruit and vegetable waste; the method comprises a primary fermentation stage and a secondary fermentation stage:
the primary fermentation stage is performed with zymophyte of lactic acid bacteria, at a fermentation temperature of 35 to 40 °C, a pH of 4.5 to 5.5, and an ORP in a fermentation system maintained at -250mV to -450mV by means of intermittent aeration;
at the secondary fermentation stage, aerobic fermentation is performed by using acclimated sludge, at a fermentation temperature of 22 to 28 °C and a pH of 7.8 to 8.3, wherein the secondary fermentation stage is an aerobic fermentation using continuous air aeration, at an aeration intensity of 0.8 to 1.2 L/L/min;
the preparation method of the acclimated sludge is as follows:
the bottom sludge of aerobic aeration tank in a sewage treatment plant is acclimated in a fermentation system with a continuous air aeration, and a pH of 6.5 to 9.5 at room temperature, by using acetic acid as a carbon source and ammonium chloride as a nitrogen source, an intensity of continuous air aeration is 0.8 to 1.2 L/L/min;
after acclimation, main flora in the acclimated sludge is *Paracoccus* and *Gemmobacter,* and the content of polyhydroxyalkanoate accounts for 27% to 42% of dry weight of bacterial cells;
the fermentation substrate is a mixture of livestock and poultry manure and fruit and vegetable waste, with a carbon to nitrogen ratio of the mixture of (30 to 50): 1;
or the fermentation substrate is a mixture of livestock and poultry manure and crop straw, with a carbon to nitrogen ratio of the mixture of (30 to 40): 1.

2. The method according to claim 1, wherein at the primary fermentation stage, an air aeration rate is 0 to 2000 mL/L/d; an air aeration rate of the primary fermentation stage is 0 to 400 mL/L/min, and an aeration time is 0 to 10 min/d.

3. The method according to claim 1, wherein the lactic acid bacteria comprise one or more of *Bifidobacterium, Streptococcus thermophilus, Lactobacillus plantarum, Lactobacillus acidophilus, Lactobacillus rhamnosus, Lactobacillus bulgaricus.*

4. The method according to claim 3, wherein at a primary fermentation stage, a fermentation concentration is 2% to 15% VS; an inoculation amount of the bacterial broth is 2% to 10% V/V, and the number of live bacteria in the bacterial broth is 10⁶ to 10⁹ CFU/mL;
and/or, at a secondary fermentation stage, a fermentation concentration is 60 to 120 mmol C/L; an inoculation amount of acclimated sludge is 40% to 80% V/V, and a hydraulic retention time is 5 to 36 hours.
